# EUROPEAN PATENT APPLICATION

(11) **EP 4 668 283 A1**
(43) Date of publication of application: **24.12.2025**
(21) Application number: 25183209.3
(22) Date of filing: 17.06.2025
(51) Int. Cl.: G16C 60/00, G16C 20/70, G16C 20/30

(54) **METHOD FOR GENERATING RECIPE OF POLYMER COMPOSITE MATERIAL AND DEVICE THEREFOR**

(30) Priority: 21.06.2024 KR 20240081274
(71) Applicant: SK innovation Co., Ltd., Seoul 03188 (KR); SK Geo Centric Co., Ltd., Seoul 03188 (KR)
(72) Inventor: JEON, Chan Woong, 34124 Daejeon (KR); KWON, June Haan, 34124 Daejeon (KR); KIM, Duk Hee, 34124 Daejeon (KR); KIM, Seok Jun, 34124 Daejeon (KR); KIM, Sun Ryong, 34124 Daejeon (KR); KIM, Jeong Hwan, 34124 Daejeon (KR); KIM, Chan Woo, 34124 Daejeon (KR); LEE, Joo Pyung, 34124 Daejeon (KR); LEE, Hee Eun, 34124 Daejeon (KR); JUNG, Hyun Jung, 34124 Daejeon (KR)
(74) Representative: Lorenz Seidler Gossel Part. mbB

(57) **Abstract**

According to various embodiments, a method for generating recipes of a polymer composite material, which includes: acquiring a prediction recipe based on a preset target property of a target polymer composite material and a recipe prediction model; acquiring a result property of a polymer composite material generated based on the prediction recipe; calculating a difference value between the target property and the result property; and outputting or modifying the prediction recipe based on a result of comparing the difference value with a preset reference value, and a device thereof may be provided.

## Description

### [CROSS-REFERENCE TO RELATED APPLICATION]

The present application claims priority to and the benefit of Korean Patent Application No. 10-2024-0081274, filed on June 21, 2024, in the Korean Intellectual Property Office.

### [BACKGROUND OF THE INVENTION]

### 1. Field of the Invention

The present disclosure relates to a method for generating recipes of a polymer composite material and a device thereof.

### 2. Description of the Related Art

A polymer composite material is used in various industrial fields, and in order to predict properties according to recipes, or predict materials of a polymer composite material having target properties during the development and synthesis thereof, experiments, simulations, and data-based approaches are comprehensively utilized.

The current techniques use methods that still require repeated trial and error in order to predict materials for the polymer composite material with target properties, for example, a method of collecting property data by trying the prediction while changing various composition ratios and process conditions in a laboratory, inferring the properties of the material based on the collected property data, and improving the mixing of materials to secure a composition of the materials is used.

Thereby, attempts are being made to predict materials for the polymer composite material with target properties using a model trained based on the artificial intelligence currently developed, but it takes a lot of costs and time to perform training of the commercially available artificial intelligence model, and in particular, there are limitations due to a reason that it is difficult to consider numerous variables and interactions between recipes and properties of the material synthesized based on the recipes.

### [SUMMARY OF THE INVENTION]

It is an object of the present disclosure to provide a method for generating recipes of a polymer composite material and a device thereof.

Problems to be solved through various embodiments are not limited to the above-described problem, and other problems not described above will be clearly understood by those skilled in the art from the following description.

To achieve the above object, according to an aspect of the present invention, there is provided a method for generating recipes for a polymer composite material, the method including: acquiring a prediction recipe based on a preset target property of a target polymer composite material and a recipe prediction model; acquiring a result property of a polymer composite material generated based on the prediction recipe; calculating a difference value between the target property and the result property; and outputting or modifying the prediction recipe based on a result of comparing the difference value with a preset reference value.

Here, the prediction recipe may include two or more materials including at least one polymer and mixing ratios for each of the two or more materials.

Here, the acquiring of a prediction recipe and the modifying of the prediction recipe may determine the mixing ratios so that a content of at least one material of the two or more materials satisfies a preset ratio.

Here, the two or more materials may include a recycled polymer produced using waste plastic as a raw material.

Here, the recycled polymer may include at least one of recycled polypropylene (PP), recycled polyethylene (PE), or recycled polyethylene terephthalate (PET).

Here, the step of outputting or modifying the prediction recipe may include: comparing the difference value with the preset reference value; if the difference value exceeds the preset reference value, modifying the prediction recipe based on the recipe prediction model; and if the difference value is the preset reference value or less, outputting the prediction recipe used for calculating the difference value.

Here, the step of modifying the prediction recipe may include: modifying the recipe prediction model by inputting the target property, the prediction recipe, and the result property into the recipe prediction model; acquiring a modified recipe based on the modified recipe prediction model and the target property; and outputting the modified recipe as the prediction recipe to be acquired.

Here, the step of acquiring a result property may include: acquiring a polymer composite material generated based on the prediction recipe; and measuring properties of the polymer composite material generated based on the prediction recipe to acquire the result property.

Here, the recipe prediction model may be generated through acquiring a plurality of learning recipes which include two or more learning materials including at least one polymer and mixing ratios for each of the two or more learning materials, and properties of a plurality of learning polymer composite materials according to each of the plurality of learning recipes as a dataset; and the recipe prediction model may be trained based on the dataset, and when inputting the target property, may be generated through the step of training to predict a recipe of a polymer composite material which satisfies the input target property.

Here, the recipe prediction model may be configured based on an optimization algorithm, and may be configured to set properties of the polymer composite material, which are an output of the property prediction model, as an output variable to be maximized or minimized, and predict a recipe, which is an input variable of the property prediction model.

Here, the target property and the result property may include at least one item of melt index, tensile strength, tensile failure, tearing strength, yield strength, flexural modulus, impact strength, elongation at break, heat distortion temperature, air permeability, and shrinkage, and a value for the at least one item.

According to another aspect of the present invention, there is provided a device for generating recipes for a polymer composite material, the device including: a recipe prediction unit configured to acquire a prediction recipe based on a preset target property of a target polymer composite material and a recipe prediction model; a property information processing unit configured to acquire a result property of a polymer composite material generated based on the prediction recipe, and calculate a difference value between the target property and the result property; a recipe model processing unit configured to modify the prediction recipe based on a result of comparing the difference value with a preset reference value; and a result output unit configured to output the prediction recipe based on the result of the comparison.

Here, the prediction recipe may include two or more materials including at least one polymer and mixing ratios for each of the two or more materials.

Here, the recipe prediction model may be configured to determine the mixing ratios in which a content of at least one material of the two or more materials satisfies a preset ratio.

Here, the two or more materials may include a recycled polymer produced using waste plastic as a raw material.

Here, the recycled polymer may include at least one of recycled polypropylene (PP), recycled polyethylene (PE), or recycled polyethylene terephthalate (PET).

Here, the property information processing unit may compare the difference value with the preset reference value, and if the difference value exceeds the preset reference value, determine to modify the prediction recipe based on the recipe prediction model, and if the difference value is the preset reference value or less, determine to output the prediction recipe used for calculating the difference value.

Here, the recipe model processing unit may modify the recipe prediction model by inputting the target property, the prediction recipe, and the result property into the recipe prediction model, acquire a modified recipe based on the modified recipe prediction model and the target property, and output the modified recipe as the acquired prediction recipe to modify the prediction recipe.

Here, the property information processing unit may acquire a polymer composite material generated based on the prediction recipe, and measure properties of the polymer composite material generated based on the prediction recipe to acquire the result property.

Here, the recipe model processing unit may process training of the recipe prediction model, and the recipe prediction model may acquire a plurality of learning recipes which include two or more learning materials including at least one polymer and mixing ratios for each of the two or more learning materials, and properties of a plurality of learning polymer composite materials according to each of the plurality of learning recipes as a dataset, and the recipe prediction model may be trained based on the dataset, and when inputting the target property, may be trained to predict a recipe of a polymer composite material which satisfies the input target property.

Here, the recipe prediction model may be configured based on an optimization algorithm, and may be configured to set properties of the polymer composite material, which are an output of the property prediction model, as an output variable to be maximized or minimized, and predict a recipe, which is an input variable of the property prediction model.

Here, the target property and the result property may include at least one item of melt index, tensile strength, tensile failure, tearing strength, yield strength, flexural modulus, impact strength, elongation at break, heat distortion temperature, air permeability, and shrinkage, and a value for the at least one item.

According to another aspect of the present invention, there is provided a method for generating recipes for a target polymer composite material having a desired preset target property, the method comprising acquiring a prediction recipe for the polymer composite material, the prediction recipe comprising at least one virgin polymer material, at least one recycled polymer material, and a mixing ratio for the least one virgin polymer material and the at least one recycled polymer material, wherein the prediction recipe is provided using a recipe prediction model for obtaining a polymer composite material with the preset target property; acquiring a result property of the polymer composite material generated based on the prediction recipe; calculating a difference value between the target property and the result property; and outputting or modifying the prediction recipe based on a result of comparing the difference value with a preset reference value.

According to various embodiments, the method for generating recipes for a polymer composite material and the device thereof may provide a deep learning model for predicting a recipe capable of synthesizing a polymer composite material according to a target property of the target polymer composite material in response to the target property thereof, thereby providing an environment capable of quickly checking materials for synthesizing a polymer composite material having the target property and the mixing ratios thereof.

According to various embodiments, the method for generating recipes for a polymer composite material and the device thereof may provide an effective feedback loop when modifying the prediction recipe by reflecting the synthesis environment of the actual polymer composite material by modifying the prediction recipe based on the properties measured from the actual synthesized polymer composite material using the prediction recipe.

According to various embodiments, the method for generating recipes of a polymer composite material and the device thereof allow users to easily convert and check a material mixing ratio between a pure polymer and a recycled polymer by inputting the properties of the target polymer composite material, thereby significantly reducing the time and costs required for development of materials.

According to various embodiments, the method for generating recipes for a polymer composite material and the device thereof may accurately predict a recipe which satisfies target property criteria of the synthesized polymer composite material while using the recycled plastic as a raw material, thereby providing an environment capable of activating the development of materials using the waste plastic.

### [BRIEF DESCRIPTION OF THE DRAWINGS]

The above and other objects, features and other advantages of the present invention will be more clearly understood from the following detailed description taken in conjunction with the accompanying drawings, in which:
FIG. 1 is a block diagram schematically illustrating the configuration of a device according to an embodiment of the present invention;
FIG. 2 is a flowchart illustrating the flow of an operation of predicting a recipe for a polymer composite material with a target property in the device according to an embodiment;
FIG. 3 is a flowchart illustrating the detailed flow of an operation of acquiring properties of the polymer composite material synthesized according to the recipe predicted in the operation of predicting a recipe for a polymer composite material with a target property in the device according to an embodiment;
FIG. 4 is a flowchart illustrating the detailed flow of an operation of outputting or modifying the recipe predicted in the operation of predicting a recipe for a polymer composite material with a target property in the device according to an embodiment;
FIG. 5 is a diagram schematically illustrating an operation of a recipe prediction model for predicting a recipe in response to the input target property in the device according to an embodiment;
FIG. 6 is a view illustrating a part of an operation of a property prediction model which is the basis of the recipe prediction model for predicting a recipe based on the optimization algorithm in the device according to an embodiment; and
FIG. 7 is a view illustrating a part of the detailed operation of the property prediction model which is the basis of the recipe prediction model for predicting a recipe based the optimization algorithm in the device according to an embodiment.

### [DETAILED DESCRIPTION OF THE INVENTION]

Hereinafter, embodiments will be described in detail with reference to the accompanying drawings. However, since various changes may be made in the embodiments, the scope of the patent invention is defined by the claims and thus, not limited or restricted by these embodiments..

It will be understood that when a component is described to as being "connected," "combined" or "coupled" to another component, the component may be directly connected or coupled the another component, but it may be "connected," "combined" or "coupled" to the another component intervening another component may be present.

Further, in describing the components of the embodiment, the meaning of "or" may mean each of the components, may mean two or more of the components, or may mean all of the components. For example, it should be understood that the expressions "a, b or c" represent any one of "a," "b," "c," "a and b," "a and c," "b and c," and "a, b and c."

Components included in one embodiment and components including common functions will be described using the same names in other embodiments. The description given in one embodiment may be applied to other embodiments, and therefore will not be described in detail within the overlapping range, unless there is a description opposite thereto.

The device and/or 'data' processed by the device may be expressed in terms of 'information'. Here, the information may be used as a concept including the data.

The present disclosure provides a method for generating recipes for a polymer composite material and a device thereof, which will be described below. To describe in more detail, the present disclosure relates to a method for generating recipes for a polymer composite material corresponding to target property and a device thereof, and may provide a method for generating recipes for a polymer composite material which satisfies target property information input from a user as a minimum value or a maximum value and a device thereof, which will be described below.

According to various embodiments, an operation of generating recipes for a polymer composite material may be performed based on at least one deep learning algorithm. To describe in more detail, the operation of generating recipes for a polymer composite material may be performed based on at least one deep learning model.

The deep learning model may include a recipe prediction model configured to predict materials containing at least one polymer and a mixing ratio of the materials so as to satisfy the input target property.

Here, the recipe prediction model may include at least one objective function generated based on at least one machine learning algorithm (or machine learning optimization algorithm). In this case, the machine learning algorithm (or a machine learning optimization algorithm) may include at least some of optimization algorithms based on machine learning, such as a Bayesian optimization algorithm, a grid search algorithm, and a gradient descent algorithm, etc.

Here, the polymer composite material may be a material prepared using at least one polymer, as well as polymer blends, polymer copolymer, polymer nanocomposites, polymer interpenetrating network (IPN), or polymer metal composites.

In addition, the polymer which is the material of the polymer composite material may include at least one polymer among polypropylene (PP), polyethylene (PE), and polyethylene terephthalate (PET).

In this case, the polymer may include polypropylene, polyethylene, and polyethylene terephthalate as polymer categories, and may include at least one polymer material for each of these categories.

Hereinafter, various embodiments of the present disclosure will be described with reference to the accompanying drawings. However, the drawings attached to the present specification serve to further understand the technical idea together with the detailed description, such that the present disclosure should not be construed as being limited only to the illustrations of the drawings.

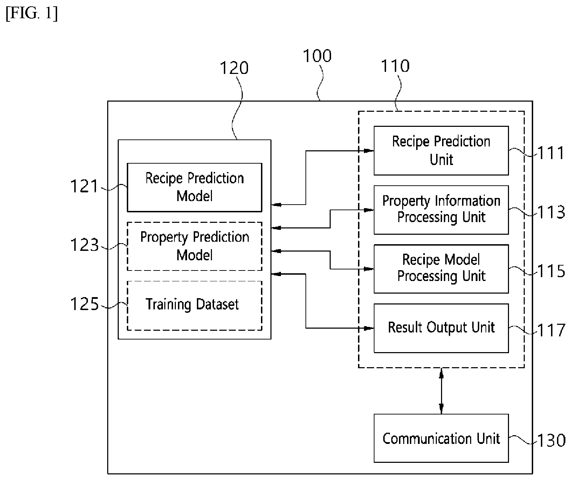

FIG. 1 is a block diagram schematically illustrating the configuration of a device according to an embodiment of the present invention. To describe in more detail, the device may be illustrated as a block diagram by dividing the detailed configuration according to functions thereof as shown in FIG. 1.

First, referring to FIG. 1, a device 100 may include at least some of a processing unit 110, a storage unit 120, and a communication unit 130.

The processing unit 110 includes at least one processor, and may process data received from an apparatus for preparing a polymer composite material connected to the device 100 through at least one program (application, tool, plug-in, software, etc., hereinafter referred to as recipe prediction program).

The processing unit 110 may include: a recipe prediction unit 111 configured to acquire a prediction recipe based on at least one target property for a target polymer composite material to be synthesized and a recipe prediction model 121, in order to predict a recipe capable of synthesizing (or compounding) a polymer composite material with a target property using the input properties; a property information processing unit 113 configured to acquire properties of the polymer composite material generated based on the prediction recipe; a recipe model processing unit 115 configured to modify the prediction recipe based on the generated properties of the polymer composite material; and a result output unit 117 configured to output the prediction recipe.

Here, it is illustrated and described that the recipe prediction unit 111, the property information processing unit 113, the recipe model processing unit 115, and the result output unit 117 are included in the processing unit 110, but they are not limited thereto, and at least some of the recipe prediction unit 111, the property information processing unit 113, the recipe model processing unit 115, and the result output unit 117 may be included in the device 100 as a separate component from the processing unit 110.

To describe in more detail, two or more of the recipe prediction unit 111, the property information processing unit 113, the recipe model processing unit 115, and the result output unit 117 may be formed as one module, or each unit may be formed as a separate module. In this case, each module may include at least one processor.

Hereinafter, the functions of various embodiments described as being performed by the device 100 may be: operations processed through the recipe prediction unit 111, the property information processing unit 113, the recipe model processing unit 115, or the result output unit 117; operations processed through a module including at least some of the recipe prediction unit 111, the property information processing unit 113, the recipe model processing unit 115, and the result output unit 117; or operations processed through the processing unit 110, which may be performed by being organically connected with components of the device 100 or other devices connected to the device 100.

The storage unit 120 may store various data processed by at least one component of the device 100 (e.g., the processing unit 110, the communication unit 130 or the like). The data may include, for example, a program for processing control commands, data processed through the program, or input data and output data related thereto.

To describe in more detail, the storage unit 120 may include an artificial algorithm for control command processing, which includes at least some of an artificial neural network algorithm, a blockchain algorithm, a deep learning algorithm, and a regression analysis algorithm based on at least some of the mechanisms, operators, language models, and big data related thereto.

The storage unit 120 may include the recipe prediction model 121 configured to predict materials including at least one polymer for generating (e.g. compounding) a polymer composite material which satisfies the input target property and a mixing ratio of the materials.

According to an embodiment, the recipe prediction model 121 may be composed of a deep learning model trained to predict a recipe (e.g., output the recipe as an output variable) which satisfies the input target property (e.g., the target property input as an input variable), for the properties and recipe of the polymer composite material.

Here, the recipe of the polymer composite material (e.g., the prediction recipe which is predicted through the recipe prediction model 121) may include two or more materials including at least one polymer for synthesizing a polymer composite material and mixing ratios for each of the two or more materials.

To this end, the storage unit 120 may include a training dataset 125 for training the recipe prediction model 121.

Here, the training dataset 125 may include learning recipes and learning property information for at least one learning polymer composite material. Here, the learning recipes may include materials including at least one polymer so as to satisfy each property of at least one learning polymer composite material and mixing ratio information of the materials, as described above through the predicted recipe.

According to various embodiments, the recipe prediction model 121 may be configured to use (or include) a pre-trained property prediction model and an optimization algorithm. For example, the recipe prediction model 121 may be configured to set properties of the polymer composite material, which are an output of the pre-trained property prediction model, as an output variable to be maximized or minimized, and predict a recipe, which is an input variable of the property prediction model.

The communication unit 130 may support establishment of a wired communication channel or establishment of a wireless communication channel inside the device 100 and/or between the device 100 and at least one other device (e.g., the user device or a server), and performing communication through the established communication channel.

The input/output unit may include or be connected to at least some of an input unit (not shown) for inputting data, such as a keyboard, mouse, or touch pad, and an output unit (not shown) for outputting data, such as a display unit (e.g., display), speaker, or driving unit.

According to various embodiments of the present invention, the device 100 or user device may include at least some of the functions in the range of all information and communication devices, including a mobile terminal, a multimedia terminal, a wired terminal, a fixed terminal, and an internet protocol (IP) terminal.

The device 100 is a device for processing the control commands, and may include at least some of the functions of a workstation or a large-scale database, or may be connected therewith through communication.

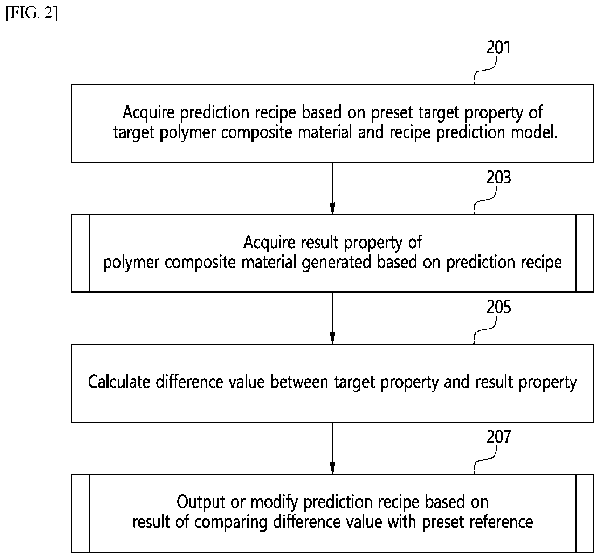

Hereinafter, as an operation of the device 100 based on FIG. 1, an operation of the device 100 to predict a recipe for synthesizing a polymer composite material which satisfies preset properties will be described with reference to FIGS. 2 to 7.

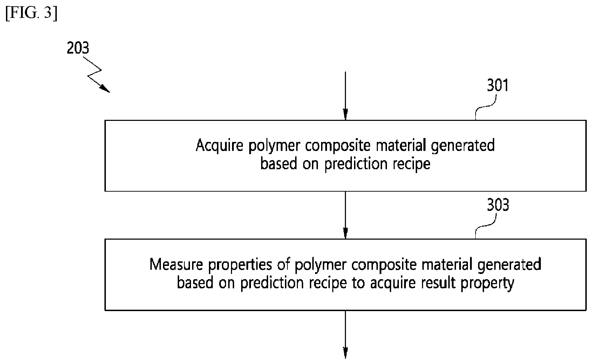

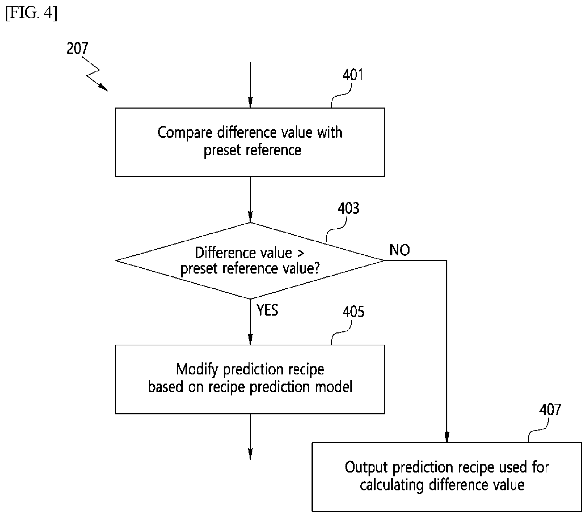

To this end, FIG. 2 is a flowchart illustrating the flow of an operation of predicting a recipe for a polymer composite material with a target property in the device according to an embodiment. FIG. 3 is a flowchart illustrating the detailed flow of an operation of acquiring properties of the polymer composite material synthesized according to the recipe predicted in the operation of predicting a recipe for a polymer composite material with a target property in the device according to an embodiment. FIG. 4 is a flowchart illustrating the detailed flow of an operation of outputting or modifying the recipe predicted in the operation of predicting a recipe for a polymer composite material with a target property in the device according to an embodiment.

First, referring to FIG. 2, in step 201, the recipe prediction unit 111 may acquire a prediction recipe based on a preset target property of the target polymer composite material and the recipe prediction model.

According to an embodiment, when the preset target property of the target polymer composite material is input, the recipe prediction unit 111 may output a recipe capable of synthesizing the target polymer composite material in response to the target property input into the recipe prediction model 121 configured to predict a recipe capable of synthesizing the target polymer composite material.

First, the recipe prediction unit 111 may acquire at least one property for the aimed polymer composite material (target polymer composite material). To describe in more detail, the recipe prediction unit 111 may acquire target property values for at least one target property item for the target polymer composite material to be synthesized.

For example, at least one target property may include at least one item of melt index, tensile strength, tensile failure, tearing strength, yield strength, flexural modulus, impact strength, elongation at break, heat distortion temperature, air permeability, and shrinkage, and values for each of the items.

To describe in more detail, the impact strength may include properties of dart impact strength and/or Izod impact strength. In addition, at least some of the tearing strength, yield strength, tensile break, and elongation at break may include properties in a machine direction and/or properties in a transverse direction.

In describing embodiments of the present invention, when expressing the 'properties (or at least one property) of a specific material', it may be expressed including properties (or property items) and property values of the specific material such as property items (e.g., tensile strength) and property values (tensile strength value possessed by a specific polypropylene) of the specific material (e.g., specific polypropylene).

According to an embodiment, the values for each of the acquired at least one property item may be acquired as a maximum value or a minimum value. For example, the recipe prediction unit 111 may acquire at least one property including a minimum value of 25 MPa in the tensile strength item, a minimum value of 0.2 g/10 min in the melt index item, a minimum value of 80 degrees Celsius (°C) in the heat distortion temperature item, and a minimum value of 1.5% in the shrinkage item.

According to the description, it is described that the recipe prediction unit 111 acquires at least one property including minimum values for tensile strength, melt index, heat distortion temperature and shrinkage.

However, it is not limited thereto, and the recipe prediction unit 111 may acquire the target property by including a maximum value for at least one item, subtracting a property value for at least one item, and/or adding the property value (maximum value or minimum value) for at least one item.

The recipe prediction unit 111 may predict a recipe (prediction recipe) for synthesizing (or capable of synthesizing) the target polymer composite material based on the at least one target property and the recipe prediction model 121. Here, the prediction recipe may include two or more materials including at least one polymer and mixing ratios for each of the two or more materials.

Here, the material includes a polymer, and may further include at least some of talc or a polyolefin elastomer.

Here, the mixing ratio is a relative weight (or mass) ratio for each of the materials (e.g., a weight ratio sum (WRS)), and a content for each material may be represented as a percent (%).

In addition, the recipe prediction unit 111 may acquire a prediction recipe including at least some of the property (material property) items for each of the included materials and property values (material property values) for the properties (material properties).

To describe in more detail, the recipe prediction unit 111 may input at least one acquired property into the recipe prediction model 121, and acquire two or more materials including at least one polymer and mixing ratios for each of the two or more materials from the recipe prediction model 121 so that the input at least one property is maximized and/or minimized as preset therein.

According to the description, that is, the recipe prediction unit 111 may process two or more property items included in the target property and the property values for each of the two or more property items as a maximum value and/or a minimum value as an input to the recipe prediction model 121, and may acquire a prediction recipe including two or more materials and mixing ratios for each of the two or more materials from the recipe prediction model 121.

The prediction recipe may include materials capable of generating a polymer composite material which satisfies at least some of the properties acquired as described above and a mixing ratio of the materials among various materials for generating a polymer composite material, such as at least one polymer, a reinforcing agent (e.g., talc), and an additive (e.g., a polyolefin ether).

To this end, the storage unit 120 may store various materials that can be used for synthesizing a polymer composite material, and information (e.g., property information) on the materials as a database.

Here, the polymer may include polypropylene (PP), polyethylene (PE), or polyethylene terephthalate (PET).

In addition, at least some of the two or more materials included in the recipe output from the recipe prediction model 121 may include identification information (e.g., code, ID, etc.) of the corresponding materials and/or a property value for at least one property item.

Here, the recipe prediction model 121 may generate a prediction recipe including identification information and/or property values about at least one property item for at least some of two or more materials included in the prediction recipe, in order to specify any one material among various materials having identical or similar properties.

For example, the polymer may include at least one of a variety of recycled polymers, such as recycled polypropylene (rPP), recycled polyethylene (rPE), and recycled polyethylene terephthalate (rPET), as well as normal or virgin polymer.

In this case, even if it is the same polymer, there may be slight differences in properties depending on the production time, etc. In particular, in the case of the recycled polymers, which are produced by reprocessing polymers acquired from various plastic wastes (or polymer wastes, waste plastics) collected such as waste plastic, waste fiber, waste rubber, waste home appliances, and waste furniture, may have a difference in the properties depending on the production time, etc., while they are not uniform.

In order to distinguish between identical materials when there is a difference in the properties, the recipe may include identification information for materials including polymers and/or property values for at least one property item that can represent characteristics of the materials.

According to various embodiments, the recipe prediction model 121 may be configured to determine a mixing ratio in which the content of at least one material among two or more materials included in the prediction recipe satisfies a preset ratio.

For example, when information on an essential polymer (e.g., at least one polymer and minimum usage ratios for each of the polymers) (essential polymer information) is input together with the target property, the recipe prediction model 121 may be configured (or trained) to output the prediction recipe (e.g., materials and mixing ratio of the materials) which includes the essential polymer in the prediction recipe and satisfies the minimum usage ratios for each of the essential polymers.

To this end, the recipe prediction model 121 may include at least one relational expression for calculating a difference in properties between the pure polymer and the recycled polymer for at least one identical polymer.

To describe in more detail, the recipe prediction model 121 may be configured to, when inputting specific polymer information set to include a specific ratio of the recycled polymer is confirmed, generate a prediction recipe composed of pure polymers based on the target property, and generate and output a prediction recipe which satisfies the essential polymer information based on the relational expression.

However, it is not limited thereto, and when the target property and essential polymer information are input, the recipe prediction model 121 may be trained to generate and output the prediction recipe which satisfies the essential polymer information.

As described above, the recipe prediction unit 111 may acquire at least some of the preset target properties and essential polymer information to input them into the recipe prediction model 121, and acquire (or output) a prediction recipe for synthesizing a polymer composite resin which satisfies the input information.

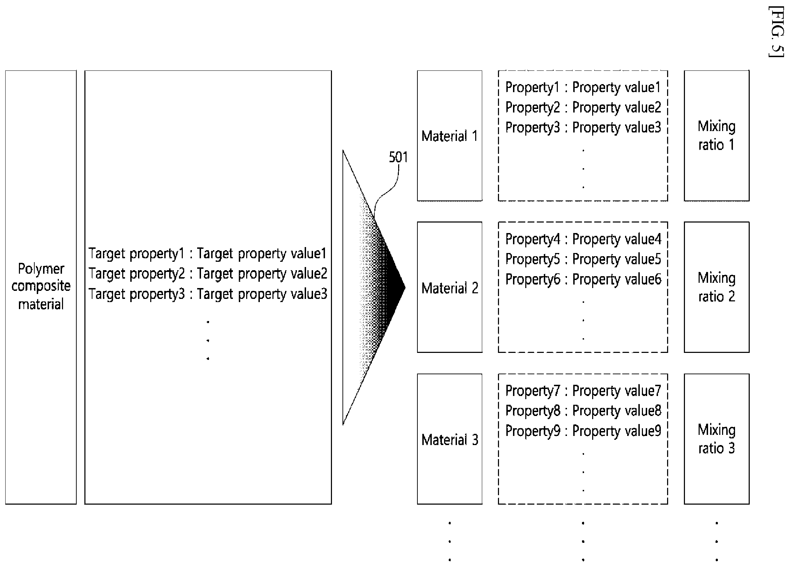

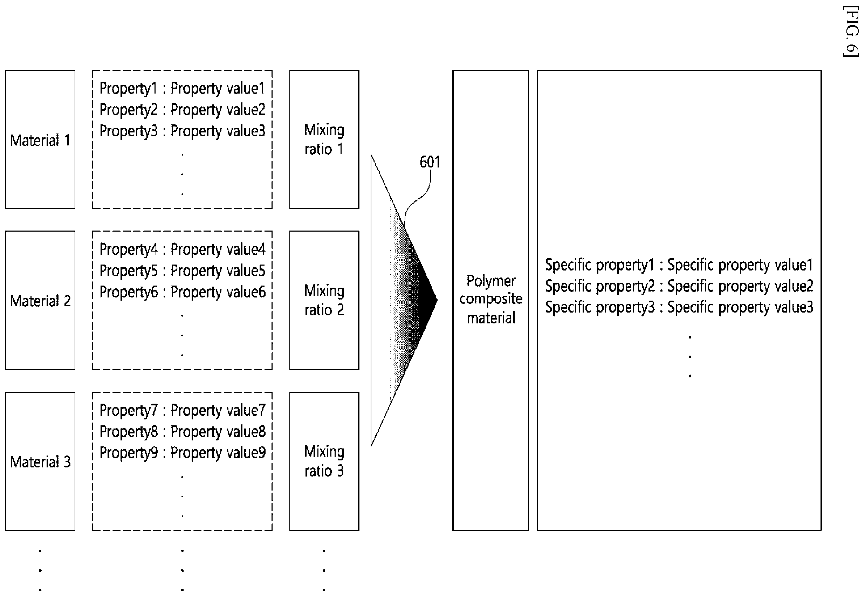

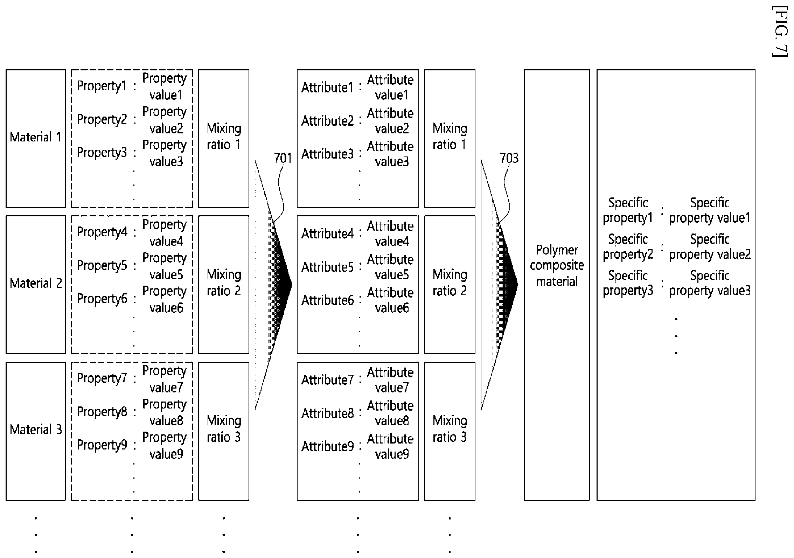

Hereinafter, the recipe prediction model 121 will be described in more detail with reference to FIGS. 5 to 7. In this regard, FIG. 5 is a diagram schematically illustrating an operation of a recipe prediction model for predicting a recipe in response to the input target property in the device according to an embodiment. FIG. 6 is a view illustrating a part of an operation of a property prediction model which is the basis of the recipe prediction model for predicting a recipe based on the optimization algorithm in the device according to an embodiment. In addition, FIG. 7 is a view illustrating a part of the detailed operation of the property prediction model which is the basis of the recipe prediction model for predicting a recipe based the optimization algorithm in the device according to an embodiment.

First, referring to FIG. 5, the operation and training of the recipe prediction model 121 including a property-recipe prediction model 501 trained to predict a recipe in response to the input target property will be described.

The property-recipe prediction model 501 may be configured to perform training based on at least some of various deep learning algorithms which process structured data, such as TabNet, XGBoost, LightGBM, CatBoost, and deep neural network (DNN).

Based on the above-described contents with respect to FIG. 5 and the recipe prediction model 121, the property-recipe prediction model 501 may be trained to receive input of at least some of the properties (e.g., target property value 1 of target property 1, target property value 2 of target property 2, target property value 3 of target property 3, etc.) of a target polymer composite material to be synthesized as the target property, and predict a recipe including a plurality of materials (e.g., material 1, material 2, and material 3, etc.) capable of synthesizing a target polymer composite material corresponding to the target property, properties of each of the materials (e.g., properties including property value 1 for property 1, property value 2 for property 2, and property value 3 for property 3 in material 1, properties including property value 4 for property 4, property value 5 for property 5, and property value 6 for property 6 in material 2, and properties including property value 7 for property 7, property value 8 for property 8, and property value 9 for property 9 in material 3), and mixing ratios for the plurality of materials (e.g., mixing ratio 1 for material 1, mixing ratio 2 for material 2, and mixing ratio 3 for material 3).

Here, it has been described that the target polymer composite material includes three target property items, but at least some of the number of target property items included in the target polymer composite material may be changed depending on the setting.

In addition, it has been described that the prediction recipe which is predicted for the synthesis of the target polymer composite material includes three materials and three property items for each of the three materials, but at least some of the number of materials included in the prediction recipe and the number of property items for each of the materials may be changed depending on the setting.

In order to train the property-recipe prediction model 501, the recipe model processing unit 115 may acquire the training dataset 125 including a plurality of recipes and properties of the polymer composite material according to the recipe for each of the plurality of recipes. Here, each of the plurality of recipes may include at least some pieces of information on two or more materials, properties of each of the two or more materials, and mixing ratios of each of the two or more materials.

To describe in more detail, the recipe model processing unit 115 may acquire two or more learning materials including at least one polymer, a plurality of learning recipes including mixing ratios for each of the two or more learning materials, and properties of a plurality of learning polymer composite materials according to each of the plurality of learning recipes as the dataset.

Here, by using information on any one learning recipe and properties of a learning polymer composite material according to the any one learning recipe, the learning recipes included in the training dataset and the learning polymer composite material corresponding to each of the learning recipes will be described.

For example, the training dataset may include learning materials (e.g., material 1, material 2, and material 3) included in a particular learning recipe, properties for the learning materials (e.g., properties including property value 1 for property 1, property value 2 for property 2, and property value 3 for property 3 in material 1, properties including property value 4 for property 4, property value 5 for property 5, and property value 6 for property 6 in material 2, and properties including property value 7 for property 7, property value 8 for property 8, and property value 9 for property 9 in material 3), and mixing ratios for the learning materials (e.g., mixing ratio 1 for material 1, mixing ratio 2 for material 2, and mixing ratio 3 for material 3).

A specific learning polymer composite material according to the specific learning recipe is a polymer composite material (e.g., a polymer composite material synthesized according to the recipe) which is confirmed to be capable of being synthesized using the specific learning recipe, and may include properties possessed by the specific learning polymer composite material (e.g., properties including polymer property value 1 for polymer property 1, polymer property value 2 for polymer property 2, and polymer property value 3 for polymer property 3).

Here, it is described that the number of materials included in the learning recipe, the number of properties included in each of the materials, and the number of properties of the learning polymer composite material are three, respectively, but they are not limited thereto, and may be changed depending on the setting.

Here, the properties of the polymer composite material may include at least some of melt index, tensile strength, tensile failure, tearing strength, yield strength, flexural modulus, impact strength, elongation at break, heat distortion temperature, air permeability, and shrinkage.

The recipe model processing unit 115 may perform training of the property-recipe prediction model 501 which is trained based on the properties of the learning materials included in each of the plurality of learning recipes, and the properties of the plurality of learning polymer composite materials, and when a target property (and/or essential polymer information) is input, predicts a recipe capable of synthesizing the polymer composite material which satisfies the target property.

The recipe model processing unit 115 may perform training of the property-recipe prediction model 501 by setting the properties of the learning polymer composite material as the target property and setting the learning recipes as the prediction recipe.

Here, when the essential polymer information is input together with the target property, the property-recipe prediction model 501 may be trained to generate a recipe which satisfies the essential polymer and the minimum content of the essential polymer.

The recipe prediction unit 111 may acquire a prediction recipe for the target property (and/or essential polymer information) using the recipe prediction model 121 including the property-recipe prediction model 501 trained as described above.

According to the description, the recipe prediction unit 111 may acquire a prediction recipe for the target property through the recipe prediction model 121 including the property-recipe prediction model 501 pre-trained to receive input of the target property and output the prediction recipe.

In addition, the recipe prediction unit 111 may also acquire the prediction recipe for the target property through the recipe prediction model 121 which is based on a property prediction model 123 pre-trained to receive input of a recipe (e.g., properties of the recipe) as an input variable and output the properties of the polymer composite material to be synthesized according to the recipe as an output variable, and includes an optimization algorithm configured to output a prediction recipe as the input variable when inputting the target property as the output variable.

Hereinafter, with reference to FIG. 6 and FIG. 7, the operation and training of the property prediction model 123 configured to predict properties of the polymer composite material to be synthesized according to the recipe in response to the input recipe (properties of the recipe), and the recipe prediction model 121 including the optimization algorithm generated based on the property prediction model 123 will be described.

First, referring to FIG. 6, the property prediction model 123 may include a property-property prediction model 601 which is trained to predict properties of the polymer composite material to be generated in response to the input recipe when the recipe is input.

Based on the above-described contents in relation to FIG. 6 and the recipe prediction model 121, the property-property prediction model 601 may be trained to receive input of materials (e.g., material 1, material 2, and material 3, etc.) and mixing ratio (e.g., mixing ratio 1 for material 1, mixing ratio 2 for material 2, and mixing ratio 3 for material 3, etc.), and predict properties (e.g., specific property 1 and specific property value 1 for specific property 1, specific property 2 and specific property value 2 for specific property 2, specific property 3 and specific property value 3 for specific property 3, etc.) of the polymer composite material predicted to be synthesized according to the input materials and the mixing ratios.

In this process, when materials (e.g., material 1, material 2, and material 3, etc.) for synthesizing a polymer composite material are input, the property-property prediction model 601 may acquire properties for the materials (e.g., properties including property value 1 for property 1, property value 2 for property 2, and property value 3 for property 3 in material 1, properties including property value 4 for property 4, property value 5 for property 5, and property value 6 for property 6 in material 2, and properties including property value 7 for property 7, property value 8 for property 8, and property value 9 for property 9 in material 3), and use the properties for the materials in training thereof.

It is described that, for the properties of the polymer composite material output by the property-property prediction model 601, three specific properties are output, but it is not limited thereto, and the properties of the polymer composite material output by the property-property prediction model 601 may further include at least one more or less specific property than three.

In addition, it has been described that the recipe input for synthesizing a polymer composite material includes three materials and three property items for each of the three materials, but at least some of the number of materials included in the recipe and the number of property items for each of the materials may be changed depending on the setting.

In order to train the property-property prediction model 601, the recipe model processing unit 115 may acquire the training dataset 125 including a plurality of recipes and properties of the polymer composite material according to the recipe for each of the plurality of recipes. Here, each of the plurality of recipes may include at least some pieces of information on two or more materials, properties of each of the two or more materials, and mixing ratios of each of the two or more materials.

To describe in more detail, the recipe model processing unit 115 may acquire two or more learning materials including at least one polymer, a plurality of learning recipes including mixing ratios for each of the two or more learning materials, and properties of a plurality of learning polymer composite materials according to each of the plurality of learning recipes as the dataset.

Here, by using information on any one learning recipe and properties of a learning polymer composite material according to the any one learning recipe, the learning recipes included in the training dataset and the learning polymer composite material corresponding to each of the learning recipes will be described.

For example, the training dataset may include learning materials (e.g., material 1, material 2, and material 3) included in a particular learning recipe, properties for the learning materials (e.g., properties including property value 1 for property 1, property value 2 for property 2, and property value 3 for property 3 in material 1, properties including property value 4 for property 4, property value 5 for property 5, and property value 6 for property 6 in material 2, and properties including property value 7 for property 7, property value 8 for property 8, and property value 9 for property 9 in material 3), and mixing ratios for the learning materials (e.g., mixing ratio 1 for material 1, mixing ratio 2 for material 2, and mixing ratio 3 for material 3).

The specific learning polymer composite material according to the specific learning recipe is a polymer composite material (e.g., a polymer composite material synthesized according to the recipe) which is confirmed to be capable of being synthesized using the specific learning recipe, and may include properties possessed by the specific learning polymer composite material (e.g., properties including polymer property value 1 for polymer property 1, polymer property value 2 for polymer property 2, and polymer property value 3 for polymer property 3).

Here, it is described that the number of materials included in the learning recipe, the number of properties included in each of the materials, and the number of properties of the learning polymer composite material are three, respectively, but they are not limited thereto, and may be changed depending on the setting.

Here, the properties of the polymer composite may include at least some of melt index, tensile strength, tensile failure, tearing strength, yield strength, flexural modulus, impact strength, elongation at break, heat distortion temperature, air permeability, and shrinkage.

The recipe model processing unit 115 may perform training of the property-property prediction model 601 which is trained based on the properties of the learning materials included in each of the plurality of learning recipes, and the properties of the plurality of learning polymer composite materials, and when a recipe (materials and mixing ratios of the materials) is input, predicts properties of the polymer composite material to be synthesized according to the recipe.

The recipe model processing unit 115 may perform training of the property-property prediction model 601 based on specific properties of at least some specific materials included in the learning recipe and specific properties of the learning polymer composite material.

To describe in more detail, first, the recipe model processing unit 115 may extract, from each of the plurality of learning recipes, a first preset learning specific property among the properties possessed by each of at least one learning specific material, and a second preset learning specific property among the properties of the learning polymer composite material.

To this end, information on the properties of the materials included in the recipe and/or at least one specific property extracted from the properties of the polymer composite material may be stored in the storage unit 120.

Here, the specific material may include a polymer, talc, or a polyolefin elastomer.

In addition, the recipe model processing unit 115 may acquire a first learning specific property or a second learning specific property, which include at least some of melt index, tensile strength, tensile failure, tearing strength, yield strength, flexural modulus, impact strength, elongation at break, heat distortion temperature, air permeability, and shrinkage.

Here, the second learning specific property may include at least one property among the specific properties of the polymer composite material to be predicted through the property-property prediction model 601.

In addition, the recipe model processing unit 115 may extract the first learning specific property in different manners based on types of the learning specific material.

For example, when the learning specific material is polypropylene (PP), the recipe model processing unit 115 may extract at least one property among the melt index, flexural modulus, tensile strength, impact strength, shrinkage, and heat distortion temperature as a first feature.

When the learning specific material is polyethylene (PE), the recipe model processing unit 115 may extract at least one property among the impact strength, tearing strength, yield strength, tensile failure, and elongation at break as a second feature.

In addition, when the learning specific material is polyethylene terephthalate (PET), the recipe model processing unit 115 may extract at least one property among the air permeability, tensile strength, tear strength, and elongation at break as a third feature.

Thereafter, the recipe model processing unit 115 may perform training of the property-property prediction model 601 for each of the plurality of learning recipes based on the plurality of learning recipes including the plurality of first learning specific properties and the second learning specific properties.

To describe in more detail, the recipe model processing unit 115 may perform training of the property-property prediction model 601 to output properties including the second learning specific properties of the specific polymer composite material corresponding to the specific learning recipe by setting the first learning specific properties and/or the second learning specific properties as a region of interest, and using the specific learning recipe and the first learning specific properties of the specific learning recipe as an input.

As described above, the property prediction model 123 may include the property-property prediction model 601 configured to predict properties of the polymer composite material to be synthesized according to the recipe using the recipe (properties of the recipe) as an input.

However, as illustrated in FIG. 7, the property prediction model 123 may include a property-attribute prediction model 701 configured to predict attributes of the materials included in the recipe using the recipe as an input, and an attribute-property prediction model 703 configured to predict properties of the polymer composite material to be synthesized according to the recipe using the attributes of the materials as an input.

Referring to FIG. 7, first, the recipe model processing unit 115 may be configured to input materials (e.g., material 1, material 2, and material 3) included in the recipe, properties for the materials (e.g., properties including property value 1 for property 1, property value 2 for property 2, and property value 3 for property 3 in material 1, properties including property value 4 for property 4, property value 5 for property 5, and property value 6 for property 6 in material 2, and properties including property value 7 for property 7, property value 8 for property 8, and property value 9 for property 9 in material 3), and mixing ratios for the materials (e.g., mixing ratio 1 for material 1, mixing ratio 2 for material 2, and mixing ratio 3 for material 3), and output attributes for the materials (e.g., attributes including attribute value 1 for attribute 1, attribute value 2 for attribute 2, and attribute value 3 for attribute 3 in material 1, attributes including attribute value 4 for attribute 4, attribute value 5 for attribute 5, and attribute value 6 for attribute 6 in material 2, and attributes including attribute value 7 for attribute 7, attribute value 8 for attribute 8, and attribute value 9 for attribute 9 in material 3).

Here, the attributes of the materials may include at least one attribute of syndiotactic PP content or molecular weight, atactic PP content or molecular weight, rubber content or molecular weight, type or content of reinforcing agent, PE content or molecular weight, type or content of copolymer, long-chain branch content, acid content, density, and degree of crystallization.

Here, referring to FIG. 7, it is described that, for the properties of the polymer composite material output by the property-attribute prediction model 701, three specific properties are output, but it is not limited thereto, and the properties of the polymer composite material output by the property-attribute prediction model 701 may further include at least one more or less specific property than three.

In addition, it has been described that the recipe input for synthesizing a polymer composite material includes three materials and three property items for each of the three materials, but as described above through FIG. 6, at least some of the number of materials included in the recipe and the number of property items for each of the materials may be changed depending on the setting.

In addition, the attribute-property prediction model 703 may be configured to receive input of attributes for the materials (e.g., attributes including attribute value 1 for attribute 1, attribute value 2 for attribute 2, and attribute value 3 for attribute 3 in material 1, attributes including attribute value 4 for attribute 4, attribute value 5 for attribute 5, and attribute value 6 for attribute 6 in material 2, and attributes including attribute value 7 for attribute 7, attribute value 8 for attribute 8, and attribute value 9 for attribute 9 in material 3), and predict specific properties (e.g., specific property value 1 for specific property 1, specific property value 2 for specific property 2, and specific property value 3 for specific property 3) of the polymer composite material according to the recipe.

Here, attributes for the materials input to the attribute-property prediction model 703 may be acquired from the property-attribute prediction model 701. In addition, the attribute-property prediction model 703 may acquire a mixing ratio for the materials from the recipe input to the property-attribute prediction model 701 to process it as an input, or acquire a mixing ratio for the materials from the output of the property-attribute prediction model 701 to process it as an input.

Here, the specific property may include at least one property of melt index, tensile strength, tensile failure, tearing strength, yield strength, flexural modulus, impact strength, elongation at break, heat distortion temperature, air permeability, and shrinkage.

As described above, the property-attribute prediction model 701 may be composed of a deep learning model configured to output the properties of the materials based on the properties of the materials included in the recipe, and the attribute-property prediction model 703 may be composed of a deep learning model trained to output specific properties of the polymer composite material according to the recipe based on the attributes of the materials.

In order to train the property-attribute prediction model 701 and the attribute-property prediction model 703, the recipe model processing unit 115 may acquire the training dataset 125 including a plurality of recipes and properties of the polymer composite material according to the recipes for each of the plurality of recipes. Here, each of the plurality of recipes may include at least some pieces of information on two or more materials, properties of each of the two or more materials, and mixing ratios of each of the two or more materials.

To describe in more detail, the recipe model processing unit 115 may acquire two or more learning materials including at least one polymer, a plurality of learning recipes including mixing ratios for each of the two or more learning materials, and properties of a plurality of learning polymer composite materials according to each of the plurality of learning recipes as the dataset.

Here, by using information on any one learning recipe and properties of a learning polymer composite material according to the any one learning recipe, the learning recipes included in the training dataset and the learning polymer composite material corresponding to each of the learning recipes will be described.

For example, the training dataset may include learning materials (e.g., material 1, material 2, and material 3) included in a particular learning recipe, properties for the learning materials (e.g., properties including property value 1 for property 1, property value 2 for property 2, and property value 3 for property 3 in material 1, properties including property value 4 for property 4, property value 5 for property 5, and property value 6 for property 6 in material 2, and properties including property value 7 for property 7, property value 8 for property 8, and property value 9 for property 9 in material 3), attributes for learning materials (e.g., attributes including attribute value 1 for attribute 1, attribute value 2 for attribute 2, and attribute value 3 for attribute 3 in material 1, attributes including attribute value 4 for attribute 4, attribute value 5 for attribute 5, and attribute value 6 for attribute 6 in material 2, and attributes including attribute value 7 for attribute 7, attribute value 8 for attribute 8, and attribute value 9 for attribute 9 in material 3), and mixing ratios for the learning materials (e.g., mixing ratio 1 for material 1, mixing ratio 2 for material 2, and mixing ratio 3 for material 3).

The specific learning polymer composite material according to the specific learning recipe is a polymer composite material (e.g., a polymer composite material synthesized according to the recipe) which is confirmed to be capable of being synthesized using the specific learning recipe, and may include properties possessed by the specific learning polymer composite material (e.g., properties including polymer property value 1 for polymer property 1, polymer property value 2 for polymer property 2, and polymer property value 3 for polymer property 3).

Here, it is described that the number of materials included in the learning recipe, the number of properties included in each of the materials, and the number of properties of the learning polymer composite material are three, respectively, but they are not limited thereto, and may be changed depending on the setting.

Here, the properties of the polymer composite may include at least some of melt index, tensile strength, tensile failure, tearing strength, yield strength, flexural modulus, impact strength, elongation at break, heat distortion temperature, air permeability, and shrinkage.

The recipe model processing unit 115 may perform training of the property-attribute prediction model 701 which is trained based on the properties of the learning materials included in each of the plurality of learning recipes, and the properties of the plurality of learning polymer composite materials, and when a recipe is input, infers attributes of the materials included in the recipe.

To describe in more detail, the recipe model processing unit 115 may extract, from each of the plurality of learning recipes, a first preset learning specific property among the properties possessed by each of at least one learning specific material, and a second preset learning specific property among the properties of the learning polymer composite material.

To this end, information on the properties of the materials included in the recipe and/or at least one specific property extracted from the properties of the polymer composite material may be stored in the storage unit 120.

Here, the specific material may include a polymer, talc, or a polyolefin elastomer.

Additionally, the recipe model processing unit 115 may extract the first learning specific property or the second learning specific property, which include at least some of melt index, tensile strength, tensile failure, tearing strength, yield strength, flexural modulus, impact strength, elongation at break, heat distortion temperature, air permeability, and shrinkage.

Here, the second learning specific property may include at least one property among the specific properties of the polymer composite material to be predicted through the property-attribute prediction model 701 and the attribute-property prediction model 703.

In addition, the recipe model processing unit 115 may extract the first learning specific property in different manners based on types of the learning specific material.

For example, when the learning specific material is polypropylene (PP), the recipe model processing unit 115 may extract at least one property among the melt index, flexural modulus, tensile strength, impact strength, shrinkage, and heat distortion temperature as a first feature.

When the learning specific material is polyethylene (PE), the recipe model processing unit 115 may extract at least one property among the impact strength, tearing strength, yield strength, tensile failure, and elongation at break as a second feature.

In addition, when the learning specific material is polyethylene terephthalate (PET), the recipe model processing unit 115 may extract at least one property among the air permeability, tensile strength, tear strength, and elongation at break as a third feature.

In addition, the recipe model processing unit 115 may perform training of the property-attribute prediction model 701 to infer attributes of the learning materials included in each of the plurality of learning recipes based on the first learning specific property included in each of the plurality of learning recipes.

To describe in more detail, the recipe model processing unit 115 may perform training of the property-attribute prediction model 701 to output attributes for at least some materials including specific materials among the materials included in the specific learning recipe by setting the first learning specific properties as a region of interest, and using the specific learning recipe and the first learning specific properties of the specific learning recipe as an input.

Thereafter, the recipe model processing unit 115 may perform training of the attribute-property prediction model 703 to predict properties of the learning polymer composite material according to each of the plurality of learning recipes based on the attributes of the learning materials included in each of the plurality of learning recipes.

To describe in more detail, the recipe model processing unit 115 may perform training of the attribute-property prediction model 703 to predict second specific properties of the learning polymer composite material according to each of the plurality of learning recipes based on the attributes of the learning materials included in each of the plurality of learning recipes, and the second preset specific properties among the properties of the learning polymer composite material.

To describe in more detail, the recipe model processing unit 115 may perform training of the attribute-property prediction model 703 to output properties including the second learning specific properties of the specific polymer composite material corresponding to the specific learning recipe by setting the second learning specific properties as a region of interest, and using the specific learning recipe, the attributes for at least some materials including specific materials among the materials included in the specific learning recipe, and the second learning specific properties of the specific learning recipe as an input.

The recipe prediction model 121 configured to output the prediction recipe for the target property based on the optimization algorithm may be generated through a training process of the above-described property prediction model 123 (e.g., the property prediction model including the property-property prediction model, or the property prediction model including the property-attribute prediction model 701 and the attribute-property prediction model 703).

To describe in more detail, the recipe prediction model 121 may include an objective function generated by applying at least one machine learning algorithm in the training process of the property prediction model 123.

For example, the recipe model processing unit 115 may perform modelling of the objective function configured to receive input of at least one target property for a target polymer composite material as an output variable in the training process of the property prediction model 123, and output the recipe (prediction recipe) for synthesizing a target polymer composite material having the target property as an input variable.

When performing modeling of the objective function, the recipe model processing unit 115 may perform modelling of the objective function by evaluating and initializing the objective function using at least a portion of the training dataset 125 configured for training the property prediction model 123, and applying a Gaussian process thereto.

As described above, the recipe prediction model 121 generated based on the property prediction model 123 may include an objective function modeled to receive input of a target property and output the recipe for synthesizing a polymer composite material according to the target property. The recipe prediction model 121 may include at least a portion of the property prediction model 123 for the operation of the recipe prediction model 121.

Returning to FIG. 2 again, the recipe prediction unit 111 may acquire a prediction recipe for synthesizing a target polymer composite material which satisfies the target property from the recipe prediction model 121 by inputting the target property into the recipe prediction model 121 configured as described above.

The recipe prediction unit 111 may output the acquired prediction recipe as a candidate recipe for synthesizing a candidate polymer composite material.

In addition, the recipe prediction unit 111 may store the acquired prediction recipe in the storage unit 120 by matching it with the target property.

In step 203, the property information processing unit 113 may acquire a result property of the polymer composite material generated based on the prediction recipe (e.g., the candidate recipe).

Here, the result property of the generated polymer composite material may include at least one item of melt index, tensile strength, tensile failure, tearing strength, yield strength, flexural modulus, impact strength, elongation at break, heat distortion temperature, air permeability, and shrinkage, and values for each of the items.

According to an embodiment, the property information processing unit 113 may acquire properties (e.g., at least one property and property value) measured for the polymer composite material (e.g., the candidate polymer composite material) synthesized according to the materials and the mixing ratios included in the prediction recipe.

To describe in more detail, the property information processing unit 113 may receive properties of the candidate polymer composite material from a property measuring device (not shown) which measures the properties of the candidate polymer composite material synthesized according to the materials and the mixing ratio of the prediction recipe.

However, it is not limited thereto, and the property information processing unit 113 may acquire properties of the candidate polymer composite material by measuring the properties of the candidate polymer composite material synthesized according to the materials and the mixing ratios of the prediction recipe through the property measuring device (not shown), as shown in FIG. 3.

To describe in more detail, the property information processing unit 113 may acquire (step 301) a candidate polymer composite material synthesized according to the materials and the mixing ratios of the prediction recipe.

Here, the candidate polymer composite material may have identification information (e.g., serial number, lot number, etc.) set therein, or may be set with the information during the synthesis process. However, it is not limited thereto, and the property information processing unit 113 may set identification information to the candidate polymer composite material in the process of acquiring the candidate polymer composite material.

The property information processing unit 113 may store the identification information of the candidate polymer composite material and the previously acquired prediction recipe in the storage unit 120 by matching with each other. Here, the previously acquired prediction recipe may be stored in the storage unit 120 by matching it with the target property as described above.

Thereafter, the property information processing unit 113 may measure the properties of the candidate polymer composite material generated based on the prediction recipe to acquire a result property of the candidate polymer composite material (step 303).

Here, the property information processing unit 113 may acquire the result property of the candidate polymer composite material through the property measuring device. To this end, the device 100 may include the property measuring device for measuring the properties of the synthesized polymer composite material, or may be connected to the property measuring device outside the device 100.

Here, the property information processing unit 113 may acquire result properties including property values for each of at least one property item included in the target property, or may measure them from the candidate polymer composite material.

The property information processing unit 113 may store the measured (or acquired) properties of the candidate polymer composite material in the storage unit 120 by matching them with the identification information of the candidate polymer composite material.

In step 205, the property information processing unit 113 may calculate a difference value between the target property and the result property.

To this end, first, the property information processing unit 113 may calculate difference values between the property values of each of the corresponding properties of the target property and the result property.

According to an embodiment, the property information processing unit 113 may calculate difference values between property items included in the target properties and property values for each of the property items (e.g., target property value 1 of target property 1, target property value 2 of target property 2, target property value 3 of target property 3, etc.) and property items included in the result properties corresponding thereto and property values for each of the property items (e.g., result property value 1 of result property 1 corresponding to target property 1, result property value 2 of result property 2 corresponding to target property 2, and result property value 3 of result property 3 corresponding to target property 3, etc.).

To describe in more detail, the property information processing unit 113 may calculate a difference value (difference value 1) between target property value 1 and result property value 1, a difference value (difference value 2) between target property value 2 and result property value 2, a difference value (difference value 3) between target property value 3 and result property value 3 and the like.

However, it is not limited thereto, and the property information processing unit 113 may calculate representative values (e.g., a representative value of the target properties and a representative value of the result properties) for the property values included in the properties (e.g., the target properties and the result properties), and calculate a difference value between the representative values (e.g., a representative difference value) (a difference value between the representative value of the target properties and the representative value of the result properties).

In step 207, the recipe model processing unit 115 may modify the prediction recipe based on the result of comparing the difference value with a preset reference, or a result output unit 117 may output the prediction recipe.

To describe in more detail with reference to FIG. 4, first, the property information processing unit 113 may compare the difference value acquired from step 203 with a preset reference value.

For example, the property information processing unit 113 may compare the difference value 1 for property 1 with a preset reference value 1 for property 1 (or for the difference value 1). Similarly, the property information processing unit 113 may compare the difference value 2 for property 2 with the preset reference value 2 for property 2 (or for the difference value 2), and compare the difference value 3 for property 3 with the preset reference value 3 for property 3 (or for the difference value 3) (step 401).

However, when the difference value of the representative values is calculated, the property information processing unit 113 may compare the difference value between the representative values with preset reference values (a representative reference value) for the representative values (or for the representative difference value) (step 401).

For example, the property information processing unit 113 may determine whether the difference values for the preset property items exceed the reference values for the preset property items, or whether the representative difference value exceeds the preset representative reference value (step 403).

Here, the preset property item may include one or more specific property items depending on the setting.

When the difference value for the preset property item exceeds the reference value for the preset property item, or when the representative difference value exceeds the preset representative reference value, the recipe model processing unit 115 may modify the prediction recipe based on the recipe prediction model 121 (step 405).

To describe in more detail, the recipe model processing unit 115 may perform retraining of the recipe prediction model 121, and regenerate a prediction recipe based on the retrained recipe prediction model 121.

According to an example, the recipe prediction model 121 may include a deep learning model trained to receive input of the target property and output the result property. In this case, the recipe model processing unit 115 may retrain the recipe prediction model 121 based on the target property, at least one previously acquired prediction recipe, and at least one previously acquired result property.

Here, when two or more prediction recipes and result properties are confirmed, the recipe model processing unit 115 may perform retraining of the recipe prediction model 121 by including the prediction recipe and the result property up to a preset number of times (c) of prediction recipe modifications, including the last confirmed prediction recipe and the last confirmed result property, as a retraining dataset.

Here, it will be described by assuming that the number of prediction recipe modifications is set to be twice (c=2) to construct the retraining dataset.

For example, if the modification of the prediction recipe is performed three times, the recipe model processing unit 115 may construct the retraining dataset by including a second prediction recipe, a second result property, a third prediction recipe, and a third prediction property among an initial prediction recipe, an initial result property, a first prediction recipe (after modification of the prediction recipe once), a first result property (after modification of the prediction recipe once), the second prediction recipe (after modification of the prediction recipe twice), the second result property (after modification of the prediction recipe twice), the third prediction recipe (after modification of the prediction recipe three times), and the third prediction property (after modification of the prediction recipe three times).

The recipe model processing unit 115 may modify the prediction recipe (e.g., perform step 201) by inputting the target property into the retrained recipe prediction model 121 and outputting a fourth prediction recipe.

As described above, the recipe model processing unit 115 may modify the recipe prediction model 121 by inputting the target property, the prediction recipe, and the result property into the recipe prediction model, acquires a modified recipe based on the modified recipe prediction model and the target property, and outputs the modified recipe to the acquiring prediction recipe (of step 201) to modify the prediction recipe. The recipe model processing unit 115 may set the modified recipe of step 405 to the acquiring prediction recipe in step 201, by performing step 201 after performing step 405.

Thereafter, the fourth prediction recipe predicted by using the retrained recipe prediction model 121 and a fourth result property of the polymer composite material according to the fourth prediction recipe are acquired (e.g., perform step 203), a difference value (or a representative difference value) between the fourth result property and the target property is calculated (e.g., perform step 205), and if the difference value (or representative difference value) exceeds the preset reference, the recipe model processing unit 115 may perform retraining of the recipe prediction model 121 by constructing the retraining dataset including the third prediction recipe, the third prediction property, the fourth prediction recipe, and the fourth result property, and output a fifth prediction recipe (modification of the prediction recipe) (e.g., perform step 207) by inputting the target property into the retrained recipe prediction model 121.

Here, the preset number of times (c) for retraining of the recipe prediction model 121 may be changed depending on the setting.

Here, the retraining dataset may include at least a portion of the training dataset used for training the recipe prediction model 121.

According to another example, the recipe prediction model 121 may include the property prediction model and the optimization algorithm trained to predict properties by input the recipe.

In this case, the recipe model processing unit 115 may modify (or regenerate) the prediction recipe based on the target property, at least one previously acquired prediction recipe, at least one previously acquired result property, and the optimization algorithm which constitutes the recipe prediction model 121.

Here, when two or more prediction recipes and result properties are confirmed, the recipe prediction model 121 may modify (or regenerate) the prediction recipe by applying the prediction recipe and result properties up to the preset number of times (c) of prediction recipe modifications, including the last confirmed prediction recipe and the last confirmed result property, to the optimization algorithm of the recipe prediction model 121.

Here, it will be described by assuming that the number of prediction recipe modifications is set to be twice (c=2) to modify (or regenerate) the prediction recipe.

For example, if the modification of the prediction recipe is performed three times, the recipe model processing unit 115 may modify the prediction recipe (e.g., perform step 201) by generating a fourth prediction recipe using the second prediction recipe, the second result property, the third prediction recipe, the third prediction property, and the optimization algorithm among the initial prediction recipe, the initial result property, the first prediction recipe (after modification of the prediction recipe once), the first result property (after modification of the prediction recipe once), the second prediction recipe (after modification of the prediction recipe twice), the second result property (after modification of the prediction recipe twice), the third prediction recipe (after modification of the prediction recipe three times), and the third prediction property (after modification of the prediction recipe three times).

Thereafter, the fourth result property of the polymer composite material according to the fourth prediction recipe is acquired (e.g., perform step 203), a difference value (or a representative difference value) between the fourth result property and the target property is calculated (e.g., perform step 205), and if the difference value (or representative difference value) exceeds a preset reference, the recipe model processing unit 115 may output a fifth prediction recipe (modify the prediction recipe) (e.g., perform step 207) based on the third prediction recipe, the third prediction property, the fourth prediction recipe, the fourth result property, and the optimization algorithm of the recipe prediction model 121.

Here, the preset number of times (c) for modifying (or regenerating) the prediction recipe based on the optimization algorithm may be changed depending on the setting.

According to various embodiments, the modification of the prediction recipe based on the optimization algorithm may be the steps of, by the recipe model processing unit 115, performing retraining of the recipe prediction model 121, and regenerating a prediction recipe based on the optimization algorithm modified (or newly subjected to modeling) in the retraining process of the recipe prediction model 121.

To this end, the recipe model processing unit 115 may process retraining of the property prediction model 123 in the retraining process of the recipe prediction model 121.

When performing the step 207, if the difference value between the target property and the result property exceeds the preset reference value, the recipe model processing unit 115 may sequentially and repeatedly perform at least some operations of the operations of modifying the prediction recipe (steps 207 and 201), acquiring the result property of the polymer composite material generated based on the modified prediction recipe (step 203), calculating the difference value between the result property and the target property (205), and comparing the difference value with the preset reference value (step 207).

On the other hand, when performing the step 207, if the difference value for the preset property item is the reference value or less for the preset property item, or if the representative difference value is the preset representative reference value or less, the result output unit 117 may output the prediction recipe used for calculating the difference value (step 407). Here, the result output unit 117 may output the last acquired prediction recipe as the result recipe for synthesizing the target polymer composite material which satisfies the target property.

For example, the result output unit 117 may output the result recipe through an output unit (e.g., display) (not shown) of the device 100, or an output unit (e.g., display) of another device connected to the device 100, or may transmit the result recipe to another preset device.

At this time, the result output unit 117 may store the last acquired prediction recipe as the result recipe for the target property in the storage unit 120 by matching therewith. Here, as described above, the target property may be stored in the storage unit 120 by matching it with at least a portion of at least one previously acquired prediction recipe, and identification information of the candidate polymer composite material synthesized using the prediction recipe as the candidate recipe.

According to an embodiment, the determination of the operation of modifying (405) the prediction recipe or outputting (407) the prediction recipe based on the comparison result of the difference value and the preset reference value may be performed by the property information processing unit 113.

According to various embodiments, the result output unit 117 may generate result information (e.g., a result report) including the result recipe and the result properties (final result properties) of the result polymer composite material generated according to the result recipe, and output the result report.

To describe in more detail, the property information processing unit 113 may calculate the difference values or the representative difference value between the final result property and the target property, and calculate a property achievement rate for at least one property item.

Further, the result output unit 117 may determine an excess usage rate (e.g., an excess usage rate relative to the minimum usage rate) for at least one recycled polymer based on a minimum usage rate of the recycled polymer and the mixing ratio of the result recipe, which are set together with the target property.

The result output unit 117 may further generate result information including at least a portion of the property achievement rate for at least one property item, and the excess usage rate for at least one recycled polymer.

According to various embodiments, the result output unit 117 may include at least a portion of a formula, and/or at least a portion of a chemical structure of the polymer composite material synthesized according to the result recipe in the result information. In this case, the result output unit 117 may display a portion corresponding to the target property of the polymer composite material through highlighting or color change.

In addition, the result output unit 117 may store the information on the prediction recipe generated in the process of acquiring the result recipe, the candidate polymer composite material generated according to the prediction recipe, and the result property of the candidate polymer composite material in the storage unit 120 by matching as information on each of the candidate polymer composite materials.

To describe in more detail, the result output unit 117 may store the initial prediction recipe, the initial result property of the initial candidate polymer composite material according to the initial recipe in the storage unit 120 by matching as information on the initial candidate polymer composite material. Similarly, the result output unit 117 may store the first prediction recipe, the first result property of the first candidate polymer composite material according to the first prediction recipe in the storage unit 120 by matching as information on the first candidate polymer composite material.

When the operation of step 205 is completed, the result output unit 117 may terminate the procedures of embodiment in FIG. 2.

As described above, the method for generating recipes for a polymer composite material and the device thereof may provide a deep learning model for predicting a recipe capable of synthesizing a polymer composite material according to a target property of the target polymer composite material in response to the target property thereof, thereby providing an environment capable of quickly checking materials for synthesizing a polymer composite material having the target property and the mixing ratios thereof.

According to various embodiments, the method for generating recipes for a polymer composite material and the device thereof may provide an effective feedback loop when modifying the prediction recipe by reflecting the synthesis environment of the actual polymer composite material by modifying the prediction recipe based on the properties measured from the actual synthesized polymer composite material using the prediction recipe.

According to various embodiments, the method for generating recipes of a polymer composite material and the device thereof allow users to easily convert and check a material mixing ratio between a pure polymer and a recycled polymer by inputting the properties of the target polymer composite material, thereby significantly reducing the time and costs required for development of materials.

According to various embodiments, the method for generating recipes for a polymer composite material and the device thereof may accurately predict a recipe which satisfies target property criteria of the synthesized polymer composite material while using the recycled plastic as a raw material, thereby providing an environment capable of activating the development of materials using the waste plastic.

As described above, although the embodiments have been described with reference to the limited drawings, it will be apparent to those skilled in the art that various modifications and alternations may be applied thereto based on the various embodiments.

For example, adequate effects or results may be achieved even if the foregoing processes and methods are carried out in different order than those described above, and/or the above-described elements, such as systems, structures, devices, or circuits, are combined or coupled in different forms and modes than those described above, or substituted or switched with other components or equivalents.

In particular, when describing with reference to the flowchart, it is described that a plurality of steps are configured and the steps are sequentially executed in a designated order, but it is not necessarily limited to the designated order.

In other words, executing by changing or deleting at least some of the steps described in the flowchart or adding at least one step is applicable as an embodiment, and executing one or more steps in parallel may also be applicable as an embodiment. That is, it is not limited to that the steps are necessarily operated in a time-series order, and should be included in various embodiments of the present invention.

Therefore, other implements and other embodiments are within the scope of claims to be described below.

## Claims

1. A method for generating recipes for a polymer composite material, the method comprising:
acquiring a prediction recipe based on a preset target property of a target polymer composite material and a recipe prediction model (121);
acquiring a result property of a polymer composite material generated based on the prediction recipe;
calculating a difference value between the target property and the result property; and
outputting or modifying the prediction recipe based on a result of comparing the difference value with a preset reference value.

2. The method according to claim 1, wherein the prediction recipe comprises two or more materials including at least one polymer and mixing ratios for each of the two or more materials.

3. The method according to claim 2, wherein the acquiring of a prediction recipe and the modifying of the prediction recipe determine the mixing ratios so that a content of at least one material of the two or more materials satisfies a preset ratio.

4. The method according to claim 2 or 3, wherein the two or more materials comprise a recycled polymer produced using waste plastic as a raw material and
the recycled polymer comprises at least one of recycled polypropylene (PP), recycled polyethylene (PE), or recycled polyethylene terephthalate (PET).

5. The method according to any one of claims 1 to 4, wherein the step of outputting or modifying the prediction recipe comprises:
comparing the difference value with the preset reference value;
if the difference value exceeds the preset reference value, modifying the prediction recipe based on the recipe prediction model (121); and
if the difference value is the preset reference value or less, outputting the prediction recipe used for calculating the difference value.

6. The method according to any one of claims 1 to 5, wherein the step of modifying the prediction recipe comprises:
modifying the recipe prediction model (121) by inputting the target property, the prediction recipe, and the result property into the recipe prediction model (121);
acquiring a modified recipe based on the modified recipe prediction model (121) and the target property; and
outputting the modified recipe as the prediction recipe to be acquired.

7. The method according to any one of claims 1 to 6, wherein the step of acquiring a result property comprises:
acquiring a polymer composite material generated based on the prediction recipe; and
measuring properties of the polymer composite material generated based on the prediction recipe to acquire the result property.

8. The method according to any one of claims 1 to 7, wherein the recipe prediction model (121) is generated through acquiring a plurality of learning recipes which comprise two or more learning materials including at least one polymer and mixing ratios for each of the two or more learning materials, and properties of a plurality of learning polymer composite materials according to each of the plurality of learning recipes as a dataset; and
the recipe prediction model (121) is trained based on the dataset, and when inputting the target property, is generated through the step of training to predict a recipe of a polymer composite material which satisfies the input target property, and
wherein the recipe prediction model (121) is configured based on an optimization algorithm, and is configured to set properties of the polymer composite material, which are an output of a property prediction model (123), as an output variable to be maximized or minimized, and predict a recipe, which is an input variable of the property prediction model (123).

9. The method according to any one of claims 1 to 8, wherein the target property and the result property comprise at least one item of melt index, tensile strength, tensile failure, tearing strength, yield strength, flexural modulus, impact strength, elongation at break, heat distortion temperature, air permeability, and shrinkage, and a value for the at least one item.

10. A device for generating recipes for a polymer composite material, the device comprising:
a recipe prediction unit (111) configured to acquire a prediction recipe based on a preset target property of a target polymer composite material and a recipe prediction model (121);
a property information processing unit (113) configured to acquire a result property of a polymer composite material generated based on the prediction recipe, and calculate a difference value between the target property and the result property;
a recipe model processing unit (115) configured to modify the prediction recipe based on a result of comparing the difference value with a preset reference value; and
a result output unit (117) configured to output the prediction recipe based on the result of the comparison.

11. The device according to claim 10, wherein the prediction recipe comprises two or more materials including at least one polymer and mixing ratios for each of the two or more materials.

12. The device according to claim 11, wherein the recipe prediction model (121) is configured to determine the mixing ratios in which a content of at least one material of the two or more materials satisfies a preset ratio.

13. The device according to claim 11 or 12, wherein the two or more materials comprise a recycled polymer produced using waste plastic as a raw material, and
the recycled polymer comprises at least one of recycled polypropylene (PP), recycled polyethylene (PE), or recycled polyethylene terephthalate (PET).

14. The device according to any one of claims 10 to 13, wherein the property information processing unit (113) compares the difference value with the preset reference value, and if the difference value exceeds the preset reference value, determines to modify the prediction recipe based on the recipe prediction model (121), and
if the difference value is the preset reference value or less, determines to output the prediction recipe used for calculating the difference value, and
wherein the property information processing unit (113) acquires a polymer composite material generated based on the prediction recipe, and measures properties of the polymer composite material generated based on the prediction recipe to acquire the result property.

15. The device according to any one of claims 10 to 14, wherein the recipe model processing unit (115) modifies the recipe prediction model (121) by inputting the target property, the prediction recipe, and the result property into the recipe prediction model (121), acquires a modified recipe based on the modified recipe prediction model (121) and the target property, and outputs the modified recipe as the acquired prediction recipe to modify the prediction recipe, and
wherein the recipe model processing unit (115) processes training of the recipe prediction model (121), and
the recipe prediction model (121) acquires a plurality of learning recipes which comprise two or more learning materials including at least one polymer and mixing ratios for each of the two or more learning materials, and properties of a plurality of learning polymer composite materials according to each of the plurality of learning recipes as a dataset, and
the recipe prediction model (121) is trained based on the dataset, and when inputting the target property, is trained to predict a recipe of a polymer composite material which satisfies the input target property.
